# EUROPÄISCHE PATENTSCHRIFT

(11) **EP 2 376 911 B1**
(45) Veröffentlichungstag und Bekanntmachung des Hinweises auf die Patenterteilung: **06.03.2013**
(21) Anmeldenummer: 09796331.8
(22) Anmeldetag: 23.12.2009
(51) Int. Cl.: G01N 33/487

(54) **GEFALTETES TRÄGERBAND MIT VERBRAUCHSELEMENTEN**
FOLDED CARRIER TAPE HAVING CONSUMABLE ELEMENTS
BANDE PORTEUSE PLIÉE MUNIE D'ÉLÉMENTS CONSOMMABLES

(30) Priorität: 15.01.2009 EP 09000497
(43) Veröffentlichungstag der Anmeldung: 19.10.2011
(73) Patentinhaber: Roche Diagnostics GmbH, 68305 Mannheim (DE); F. Hoffmann-La Roche AG, 4070 Basel (CH)
(72) Erfinder: HARTTIG, Herbert, 67434 Neustadt (DE)
(74) Vertreter: Twelmeier Mommer & Partner
(86) Internationale Anmeldenummer: PCT/EP2009/009241
(87) Internationale Veröffentlichungsnummer: WO 2010/081529

(56) Entgegenhaltungen:
- WO-A-2007/077212
- WO-A-2007/147494
- WO-A2-03/045557
- US-A1- 2008 103 415

## Beschreibung

Die Erfindung geht aus von einem Trägerband mit den im Oberbegriff des Anspruchs 1 angegebenen Merkmalen. Ein derartiges Trägerband ist aus der WO 03/045557 A2 bekannt.

Ein aus der US 2008/0103415 A1 bekannte Trägerband trägt als Verbrauchselemente Lanzetten und ist zu einem Stapel gefaltet in einer Kammer eines Magazins angeordnet. Dieses Magazin bildet zusammen mit einem Stechgerät, in welches das Magazin eingesetzt werden kann, ein System zur Bestimmung einer Analytkonzentration einer menschlichen oder tierischen Körperflüssigkeit. Solche und ähnliche Systeme werden beispielsweise von Diabetikern benötigt, die mehrmals täglich ihren Blutzuckerspiegel überprüfen müssen und dazu eine Körperflüssigkeitsprobe, in der Regel Blut und/oder interstitielle Flüssigkeit, benötigen, die aus einer kleinen Stichwunde gewonnen wird.

Im Gegensatz zu Stechsystemen mit Trommelmagazinen, die typischerweise nur sechs oder acht Stechelemente enthalten, kann mit einem Trägerband ein wesentlich größerer Vorrat an Stechelementen bereitgestellt werden. Systeme mit Trägerbändern, die mehrere Verbrauchselemente tragen, haben deshalb den Vorteil, dass sich ein Benutzer seltener um den Austausch eines Trägerbandes oder, bei Verwendung von Wegwerfgeräten, um ein neues Gerät bemühen muss.

Trägerbänder können in einem Magazin auf einen Wickelkern einer Spule aufgewickelt sein, wie dies beispielsweise aus der US 2006/0173380 A1 bekannt ist. Ein solcher Wickelkern erfordert jedoch einen erheblichen Bauraum, insbesondere bei relativ breiten Verbrauchselementen, die durch einen zu engen Wickelradius beschädigt werden. Magazine mit zu einem Stapel gefalteten Trägerbändern ermöglichen demgegenüber eine bessere Ausnutzung zur Verfügung stehenden Bauraums.

Aufgabe der vorliegenden Erfindung ist es, einen Weg aufzuzeigen, wie sich mit einem Trägerband der eingangs genannten Art, das mehrere Verbrauchselemente für ein System zur Bestimmung einer Analytkonzentration trägt und zu einem Stapel gefaltet ist, die Lagerung von Verbrauchselementen in einem kompakten Volumen verbessern lässt.

Diese Aufgabe wird durch ein Trägerband mit den im Anspruch 1 angegebenen Merkmalen gelöst. Vorteilhafte Weiterbildungen der Erfindungen sind Gegenstand der Unteransprüche.

Während bei dem aus der US 2008/0103415 A1 bekannten Trägerband benachbarte Verbrauchselemente jeweils gleiche Abstände haben, sind die Verbrauchselemente eines erfindungsgemäßen Trägerbands in Gruppen angeordnet. Dabei ist zwischen benachbarten Gruppen in Längsrichtung des Trägerbandes jeweils ein größerer Abstand als zwischen benachbarten Verbrauchselementen innerhalb einer Gruppe. Unter dem Abstand zwischen benachbarten Gruppen ist dabei jeweils der Abstand zwischen dem letzten Verbrauchselement einer Gruppe und dem ersten Verbrauchselement einer darauf folgenden Gruppe zu verstehen. Das Wort benachbart bezieht sich hierbei auf die Anordnung auf einem Trägerband vor dem Falten.

Die erfindungsgemäße Anordnung der Verbrauchselemente hat den Vorteil, dass sich auch bei einer regelmäßigen Zickzack-Faltung des Trägerbandes zu einem Stapel vermeiden lässt, dass alle Verbrauchselemente übereinander liegen. Stattdessen können die Verbrauchselemente in dem erfindungsgemäßen Trägerband-Stapel zwei oder mehr nebeneinander angeordnete Verbrauchselemente-Stapel bilden. Auf diese Weise lässt sich ein günstigeres Verhältnis zwischen der Höhe des Trägerband-Stapels und seiner Länge bzw. Breite erzielen, so dass sich ein erfindungsgemäßer Trägerband-Stapel besser handhaben lässt und in einem ergonomischen Gerät ein größerer Vorrat an Verbrauchselementen gelagert werden kann.

Die Verbrauchselemente eines erfindungsgemäßen Trägerbandes können Stechelemente oder Testelemente mit Nachweisreagenzien, beispielsweise Testfelder mit Nachweisreagenzien zur photometrischen Konzentrationsbestimmung sein. Ein erfindungsgemäßes Trägerband kann beispielsweise lauter gleiche Verbrauchselemente, also Verbrauchselemente nur eines Typs, beispielsweise nur Stechelemente oder nur Testfelder, tragen oder sowohl Stechelemente als auch separate Testelemente tragen. Falls das Trägerband unterschiedliche Verbrauchselemente trägt, enthalten die einzelnen Gruppen bevorzugt jeweils mehrere Stechelemente und mehrere Testelemente. Bevorzugt ist insbesondere, dass die Verbrauchselemente als Stechelemente mit integrierten Testelementen ausgebildet sind, beispielsweise als Lanzetten mit einem Kapillarkanal zur Aufnahme von Körperflüssigkeit, der zu einem Testfeld mit Nachweisreagenzien führt.

Bevorzugt ist bei einem erfindungsgemäßen Trägerband der Abstand zwischen benachbarten Verbrauchselementen jeweils größer als die in Längsrichtung des Trägerbandes gemessene Länge des Stapels. Auf diese Weise wird erreicht, dass sich in einer Lage des Trägerband-Stapels jeweils höchstens ein einziges Verbrauchselement befindet. Zwar können Verbrauchselemente auch in dichteren Abständen auf dem Trägerband angeordnet werden, so dass sich beispielsweise in einer einziger Lage des Stapels nebeneinander zwei, drei oder noch mehr Verbrauchselemente befinden, jedoch müssen die einzelnen Verbrauchselemente dann bei der Fertigung wesentlich präziser auf dem Band positioniert werden. Größere Abstände zwischen den einzelnen Verbrauchselementen lassen demgegenüber größere Fertigungstoleranzen zu und ermöglichen deshalb eine kostengünstigere Herstellung. Besonders bevorzugt ist der Abstand zwischen benachbarten Verbrauchselementen jeweils mehr als doppelt so groß wie die in Längsrichtung des Trägerbandes gemessene Länge des Stapels. Auf diese Weise lässt sich nämlich erreichen, dass die Verbrauchselemente in dem Trägerband-Stapel in Bezug auf die Stapelrichtung einheitlich orientiert sind, also beispielsweise in Stapelrichtung stets auf dem sie tragenden Trägerbandabschnitt sitzen oder alle Verbrauchselemente jeweils unter dem sie tragenden Trägerbandabschnitt liegen. Vorteilhaft ist es insbesondere, wenn der Abstand zwischen benachbarten Verbrauchselementen jeweils mehr als die Ausdehnung eines der Verbrauchselemente in Längsrichtung des Trägerbandes zuzüglich dem doppelten der in Längsrichtung des Trägerbandes gemessenen Länge des Stapels ist. Auf diese Weise lässt sich erreichen, dass aufeinanderfolgende Verbrauchselemente in dem Trägerband-Stapel nebeneinander angeordnet sind. Die Fixierung der Verbrauchselemente auf einer Seite des Trägerbandes ist besonders vorteilhaft für die Herstellung und Anwendung.

Bevorzugt ist insbesondere, dass zwischen zwei in einem Verbrauchsmittel-Stapel übereinander liegenden Verbrauchsmitteln jeweils mindestens zwei Trägerbandlagen angeordnet sind.

Eine vorteilhafte Weiterbildung der Erfindung sieht vor, dass innerhalb der Gruppen die Abstände zwischen einem Verbrauchselement und dem nächsten gleichen Verbrauchselement jeweils gleich sind. Beispielsweise können in einer Gruppe drei Stechelemente angeordnet werden, wobei der Abstand zwischen dem ersten und dem zweiten Stechelement gleichgroß wie der Abstand zwischen dem zweiten und dem dritten Stechelement ist. Zwischen den Stechelementen können als weitere Verbrauchselemente beispielsweise Testelemente angeordnet sein, um durch Stiche gewonnene Körperflüssigkeitsproben zu untersuchen. Die Testelemente können dabei in Bezug auf die Stechelemente asymmetrisch angeordnet sein, so dass die Testelemente jeweils näher an dem Stechelement angeordnet sind, mit dem eine von dem Testelement zu untersuchende Probe gewonnen wird. Bevorzugt sind innerhalb der Gruppen die Abstände zwischen benachbarten Verbrauchselementen aber jeweils gleich. Unter gleichen Abständen sind Abstände zu verstehen, die im Rahmen der Fertigungstoleranzen gleich sind, also lediglich um Fertigungstoleranzen voneinander abweichen.

Eine weitere vorteilhafte Weiterbildung der Erfindung sieht vor, dass der Abstand zwischen benachbarten Gruppen jeweils mehr als zweimal, vorzugsweise mehr als dreimal, so groß wie die in Längsrichtung des Trägerbandes gemessene Länge des Stapels ist. Auf diese Weise können in dem Trägerband-Stapel Trägerbandschlaufen abgelegt werden, die keine Verbrauchselemente tragen. Mit derartigen Abschnitten können in einem Handgerät die Bandführung und der Bandtransport vereinfacht werden. Beispielsweise kann auf diese Weise erreicht werden, dass das Trägerband Vor- und Nachläuferabschnitte ohne Verbrauchselemente aufweist, die einen problemlosen Gebrauch des ersten sowie des letzten Verbrauchselements eines Trägerband-Stapels ermöglichen. Der Abstand zwischen benachbarten Gruppen ist bevorzugt stets gleich. Prinzipiell kann der Abstand zwischen benachbarten Gruppen aber auch variieren, insbesondere regelmäßig variieren.

Eine weitere vorteilhafte Weiterbildung der Erfindung sieht vor, dass die einzelnen Lagen des Trägerband-Stapels jeweils gleich lang sind. Ein auf diese Weise zu einem Stapel gefaltetes Trägerband wird auch als Leporello bezeichnet. Ein Leporello ist ein ziehharmonikaartig zusammengelegter Streifen. Die Faltkanten eines Leporellos verlaufen parallel zu einander senkrecht zur Längsrichtung des Streifens. Es ist jedoch auch möglich, in dem Trägerbandstapel unterschiedlich lange Lagen vorzusehen, also das Trägerband in unterschiedlichen Abständen zu falten.

Bevorzugt enthalten die Gruppen eines erfindungsgemäßen Trägerbandes jeweils 2 bis 8 Verbrauchselemente, besonders bevorzugt 3 bis 5 Verbrauchselemente. Bevorzugt ist ferner, dass das Trägerband mindestens 5 Gruppen, insbesondere mindestens 10 Gruppen trägt. Bevorzugt ist ferner, dass alle Gruppen zwischen der ersten und der letzten Gruppe eines Trägerbandes gleich sind, also dieselbe Anzahl Verbrauchselemente enthalten. Insbesondere die erste und die letzte Gruppe können abweichen, beispielsweise weniger Verbrauchselemente enthalten. Bevorzugt sind jedoch alle Gruppen gleich.

Wenn das Trägerband unterschiedliche Verbrauchselemente trägt, haben die verschiedenen Verbrauchselemente bevorzugt die gleiche Dicke, wenn eine geradzahlige Anzahl von Verbrauchselemente-Stapeln vorhanden ist. Wenn nämlich die Anzahl der Verbrauchselemente-Stapel gerade ist und zwei unterschiedliche Verbrauchselemente, beispielsweise Stechelemente und Testelemente, vorhanden sind, so liegen in einem Verbrauchselemente-Stapel stets gleiche Verbrauchselemente aufeinander. In einem solchen Fall ist es günstig, wenn die Verbrauchselemente gleich dick sind.

Ein erfindungsgemäßer Trägerbandstapel kann in einer Kammer eines Magazins angeordnet werden, das bestimmungsgemäß in ein Handgerät eingesetzt wird. Bevorzugt hat ein solches Magazin eine Transportvorrichtung, beispielsweise eine Wickeleinrichtung, um das Trägerband durch Aufwickeln aus der Magazinkammer herauszuziehen.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen bezeichnet. Es zeigen:
- Figur 1: eine schematische Darstellung eines Trägerbandes mit Verbrauchselementen;
- Figur 2: eine schematische Darstellung einer Magazinkammer mit einem darin angeordneten Trägerband-Stapel;
- Figur 3: eine schematische Darstellung eines Magazins
- Figur 4a, 4b: schematische Darstellungen zur Veranschaulichung eines Verfahrens zum Falten eines Trägerbands.

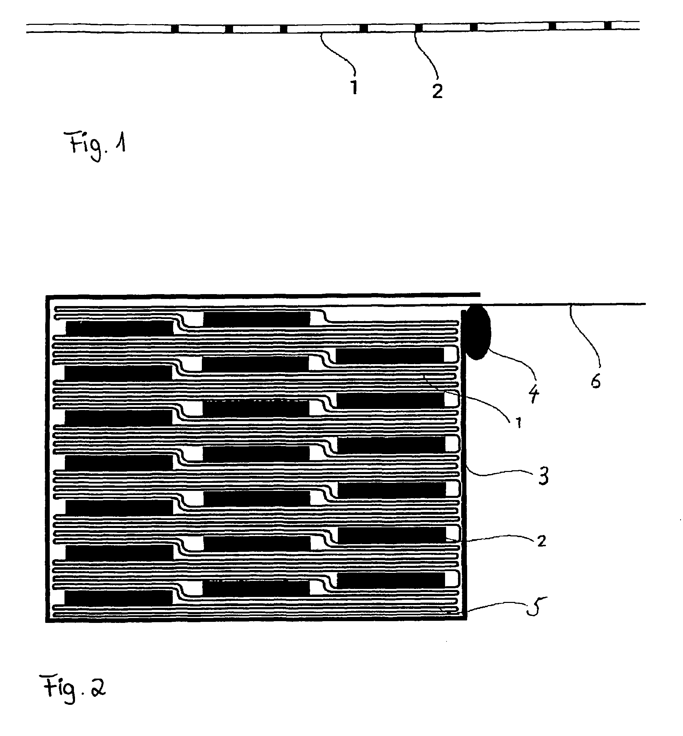

Das in Figur 1 dargestellte Trägerband 1 trägt regelmäßig angeordnete Verbrauchselemente 2, beispielsweise Stechelemente. Die Verbrauchselemente 2 sind auf dem Trägerband 1 in Gruppen angeordnet. Bei dem dargestellten Ausführungsbeispiel umfasst jede dieser Gruppen drei Verbrauchselemente 2. Zwischen benachbarten Gruppen ist dabei ein größerer Abstand als zwischen benachbarten Verbrauchselementen 2 innerhalb einer Gruppe. Bei dem dargestellten Ausführungsbeispiel sind die Abstände zwischen benachbarten Verbrauchselementen 2 innerhalb der Gruppen jeweils gleich. Ebenso sind die Abstände zwischen den einzelnen Gruppen jeweils innerhalb der Fertigungstoleranzen gleich.

Das Trägerband 1 wird zick-zack-förmig zu einem Stapel, nämlich zu einem Leporello, gefaltet und in die Kammer 3 eines Magazins eingesetzt. Figur 2 zeigt schematisch ein Ausführungsbeispiel einer Magazinkammer 3, in der ein zu einem Leporello-Stapel gefaltetes Trägerband 1 angeordnet ist. Zur Verdeutlichung sind die Lagen des Trägerbands 1 in Figur 2 mit stark überhöhtem Abstand eingezeichnet. Die einzelnen Lagen tragen dabei jeweils höchstens ein einziges Verbrauchselement 2 und haben alle die gleiche Länge.

Wie Figur 2 zeigt, bilden die Verbrauchselemente 2 in dem Trägerband-Stapel nebeneinander angeordnete Verbrauchselemente-Stapel. Bei einer regelmäßigen Zick-Zack-Faltung stimmt die Anzahl der Verbrauchselemente-Stapel mit der Anzahl der Verbrauchselemente 2 in den einzelnen Gruppen überein. Bei dem dargestellten Ausführungsbeispiel enthält der Stapel des Trägerbandes 1 folglich drei Verbrauchselemente-Stapel. Benachbarte Verbrauchselemente 2 sind dabei stets in unterschiedlichen Verbrauchslemente-Stapeln angeordnet.

In dem Stapel des Trägerbandes 1 sind alle Verbrauchselemente 2 in einer einheitlichen Orientierung angeordnet. Bei dem dargestellten Ausführungsbeispiel liegen nämlich alle Verbrauchselemente 2 jeweils in Stapelrichtung auf dem sie tragenden Trägerbandabschnitt. Dies wird dadurch erreicht, dass der Abstand zwischen Verbrauchselementen 2 innerhalb einer Gruppe jeweils mehr als doppelt so groß wie die in Längsrichtung des Trägerbandes 1 gemessene Länge des Stapels ist. Bei dem dargestellten Ausführungsbeispiel beträgt der Abstand zwischen Verbrauchselementen 2 innerhalb einer Gruppe jeweils etwas mehr als die Summe aus dem Doppelten der Länge des Trägerband-Stapels und der Länge eines Verbrauchselements 2 gemessen in Längsrichtung des Trägerbandes 1.

Bei dem dargestellten Ausführungsbeispiel enthält der Trägerband-Stapel zwischen dem letzten Verbrauchselement 2 einer Gruppe und dem ersten Verbrauchselement 2 einer darauf folgenden Gruppe eine Trägerbandschlaufe, die kein Verbrauchselement 2 trägt. Dies wird dadurch erreicht, dass der Abstand zwischen benachbarten Gruppen jeweils mehr als das Dreifache der Länge des Trägerband-Stapels ist. Bei dem dargestellten Ausführungsbeispiel beträgt der Abstand zwischen benachbarten Gruppen etwas mehr als das Vierfache der Länge des Trägerband-Stapels.

Die Magazinkammer 3 hat eine Öffnung, durch die das Trägerband 1 hindurch geführt ist. An der Öffnung der Magazinkammer 3 ist eine Durchzugsdichtung 4 angeordnet, um einem Eindringen von Schmutz oder Feuchtigkeit in die Magazinkammer 3 entgegenzuwirken. Die Durchzugsdichtung 4 kann beispielsweise als eine Lippe aus einem elastomeren Material ausgebildet sein. In Figur 2 ist zur Verbesserung der Anschaulichkeit zwischen dem Trägerband 1 und dem von der Durchzugsdichtung 4 abgewandten Rand der Öffnung der Magazinkammer 3 ein Abstand eingezeichnet. Bevorzugt wird das Trägerband 1 jedoch von der Durchzugsdichtung 4 gegen den Rand der Öffnung gedrückt. Bevorzugt ist dabei insbesondere, dass die Durchzugsdichtung 4 auf jene Seite des Trägerbandes 1 einwirkt, auf der keine Verbrauchselemente 2 angeordnet sind.

Der in die Magazinkammer 3 eingesetzte Trägerband-Stapel hat an seinem Ende einen Bandnachlauf 5 und an seinem Anfang einen Bandvorlauf 6. Auf diese Weise wird gewährleistet, dass auch das erste und das letzte Verbrauchselement 2 des Trägerbandes 1 problemlos verwendet werden können.

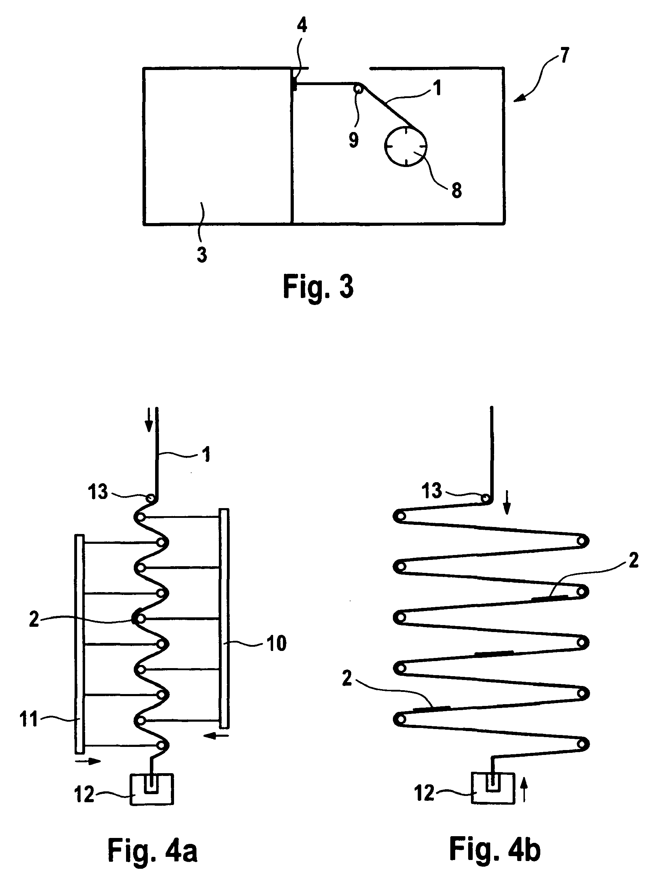

Figur 3 zeigt schematisch ein vollständiges Magazin 7 mit einer einen Trägerband-Stapel enthaltenden Magazinkammer 3 und einer Wickeleinrichtung 8 zum Aufwickeln des Trägerbands 1. Das Trägerband 1 wird aus der Magazinkammer 3 an der Durchzugsdichtung 4 vorbei über eine Umlenkung 9 zu der Wickeleinrichtung 8 geführt, an welcher der Bandvorlauf 6 des Trägerbands 1 befestigt ist. Durch Drehen der Wickeleinrichtung 8 kann das Trägerband 1 aus der Magazinkammer 3 herausgezogen werden, so dass die Verbrauchselemente 2 nacheinander verwendet werden können.

In den Figuren 4a und 4b ist schematisch dargestellt, wie ein Trägerband 1 zu einem Stapel gefaltet werden kann. Zum Falten wird das Trägerband 1 über Rollen von zwei Rollenrahmen 10, 11 geführt. Die kammartig angeordneten Rollen werden mit dem Rahmen aneinander vorbeigeschoben. Dadurch nimmt das Trägerband einen zick-zack-förmigen Verlauf. In der maximalen Distanz der Rollen wird das Trägerband 1 durch einen Greifer 12 und eine Umlenkung 13 zu einem Stapel zusammengedrückt. Die Rollen werden dann senkrecht zur Bandrichtung aus den Bandschlaufen gezogen. Nach Abtrennung des Bandzulaufs kann der gefaltete Stapel in einer Magazinkammer 3 platziert werden.

Die dargestellten Rollen können durch Umlenkstifte ersetzt werden. Ferner müssen die Rollen nicht unbedingt gleichzeitig bewegt werden, sondern können auch abwechselnd von unten nach oben bewegt werden. Anstelle der Rollenrahmen 10, 11 ist es auch möglich, eine Art großer ineinander kämmender Zahnräder zu verwenden.

Bei dem Trägerband 1 handelt es sich bevorzugt um eine Folie aus Kunststoff, beispielsweise aus Polyester. Es können jedoch auch andere Materialen, beispielsweise Papier oder Metallfolie verwendet werden.

### Bezugszahlen

- 1: Trägerband
- 2: Verbrauchselement
- 3: Magazinkammer
- 4: Durchzugsdichtung
- 5: Bandnachlauf
- 6: Bandvorlauf
- 7: Magazin
- 8: Wickeleinrichtung
- 9: Umlenkung
- 10: Rollenrahmen
- 11: Rollenrahmen
- 12: Greifer
- 13: Umlenkung

## Patentansprüche

1. Trägerband, das mehrere Verbrauchselemente (2) für ein System zur Bestimmung einer Analytkonzentration einer menschlichen oder tierischen Körperflüssigkeit trägt und zu einem Stapel gefaltet ist, wobei die Verbrauchselemente (2) auf dem Trägerband (1) in Gruppen angeordnet sind, wobei zwischen benachbarten Gruppen in Längsrichtung des Trägerbandes (1) ein größerer Abstand als zwischen benachbarten Verbrauchselementen (2) innerhalb einer Gruppe ist, **dadurch gekennzeichnet, dass** eine Lage des Stapels des Trägerbandes (1) jeweils höchstens ein einziges Verbrauchselement (2) enthält.

2. Trägerband nach Anspruch 1, **dadurch gekennzeichnet, dass** zumindest einige der Verbrauchselemente (2) Stechelemente und/oder Testelemente oder Stechelemente mit integrierten Testelementen sind.

3. Trägerband nach einem der vorstehenden Ansprüche, **dadurch gekennzeichnet, dass** jede Gruppe mindestens drei Verbrauchselemente (2) enthält.

4. Trägerband nach einem der vorstehenden Ansprüche, **dadurch gekennzeichnet, dass** der Abstand zwischen benachbarten Verbrauchselementen (2) jeweils größer als die in Längsrichtung des Trägerbandes (1) gemessene Länge des Stapels ist, vorzugsweise mindestens doppelt so groß ist.

5. Trägerband nach einem der vorstehenden Ansprüche, **dadurch gekennzeichnet, dass** der Abstand zwischen benachbarten Verbrauchselementen (2) jeweils mehr als die Ausdehnung eines der Verbrauchselemente (2) in Längsrichtung des Trägerbandes (2) zuzüglich dem Doppelten der in Längsrichtung des Trägerbandes (1) gemessene Länge des Stapels ist.

6. Trägerband nach einem der vorstehenden Ansprüche, **dadurch gekennzeichnet, dass** der Abstand zwischen benachbarten Gruppen jeweils mehr als zweimal, vorzugsweise mehr als dreimal, so groß wie die in Längsrichtung des Trägerbandes (1) gemessene Länge des Stapels ist.

7. Trägerband nach einem der vorstehenden Ansprüche, **dadurch gekennzeichnet, dass** innerhalb der Gruppen die Abstände zwischen einem Verbrauchselement (2) und dem nächsten gleichen Verbrauchselement (2) jeweils gleich sind.

8. Trägerband nach einem der vorstehenden Ansprüche, **dadurch gekennzeichnet, dass** innerhalb der Gruppen die Abstände zwischen benachbarten Verbrauchselementen (2) jeweils gleich sind.

9. Trägerband nach einem der vorstehenden Ansprüche, **dadurch gekennzeichnet, dass** die Abstände zwischen benachbarten Gruppen jeweils gleich sind.

10. Trägerband nach einem der vorstehenden Ansprüche, **dadurch gekennzeichnet, dass** die Verbrauchselemente (2) in dem Stapel mindestens zwei nebeneinander angeordnete Verbrauchselemente-Stapel bilden.

11. Trägerband nach Anspruch 10, **dadurch gekennzeichnet, dass** benachbarte Verbrauchselemente (2) stets in unterschiedlichen Verbrauchselemente-Stapeln angeordnet sind.

12. Trägerband nach einem der vorstehenden Ansprüche **dadurch gekennzeichnet, dass** die einzelnen Lagen des Stapels gleich lang sind.

13. Magazin mit einer Kammer, in der ein zu einem Stapel gefaltetes Trägerband (1) nach einem der vorstehenden Ansprüche angeordnet ist.

14. Magazin nach Anspruch 13, **gekennzeichnet durch** eine Wickeleinrichtung (8) zum Aufwickeln des Trägerbandes (1).

## Claims

1. A carrier tape which carries a plurality of consumable elements (2) for a system for determining an analyte concentration of a human or animal bodily fluid and is folded into a stack, wherein the consumable elements (2) are disposed on the carrier tape (1) in groups, wherein the distance between adjacent groups in the longitudinal direction of the carrier tape (1) is greater than that between adjacent consumable elements (2) within a group, **characterized in that** one layer of the stack of the carrier tape (1) contains one consumable element (2) at most.

2. The carrier tape according to claim 1, **characterized in that** at least a few of the consumable elements (2) are lancing elements and/or test elements or lancing elements with integrated test elements.

3. The carrier tape according to one of the preceding claims, **characterized in that** each group contains at least three consumable elements (2).

4. The carrier tape according to one of the preceding claims, **characterized in that** the distance between adjacent consumable elements (2) in each case is greater than the length of the stack as measured in the longitudinal direction of the carrier tape (1), and is preferably at least twice as great.

5. The carrier tape according to one of the preceding claims, **characterized in that** the distance between adjacent consumable elements (2) in each case is greater than the extension of one of the consumable elements (2) in the longitudinal direction of the carrier tape (2) plus twice the length of the stack as measured in the longitudinal direction of the carrier tape (1).

6. The carrier tape according to one of the preceding claims, **characterized in that** the distance between adjacent groups in each case is greater than twice, preferably greater than three times, the length of the stack as measured in the longitudinal direction of the carrier tape (1).

7. The carrier tape according to one of the preceding claims, **characterized in that** the distances between one consumable element (2) and the next identical consumable element (2) are all the same within the groups.

8. The carrier tape according to one of the preceding claims, **characterized in that** the distances between adjacent consumable elements (2) are all the same within the groups.

9. The carrier tape according to one of the preceding claims, **characterized in that** the distances between adjacent groups are all the same.

10. The carrier tape according to one of the preceding claims, **characterized in that** the consumable elements (2) in the stack form at least two consumable element stacks disposed next to one another.

11. The carrier tape according to claim 10, **characterized in that** adjacent consumable elements (2) are always disposed in different consumable element stacks.

12. The carrier tape according to one of the preceding claims, **characterized in that** the individual layers of the stack have the same length.

13. A magazine comprising a chamber in which a carrier tape (1) folded into a stack according to one of the preceding claims is disposed.

14. The magazine according to claim 13, **characterized by** a reeling device (8) for reeling the carrier tape (1).

## Revendications

1. Bande porteuse laquelle porte plusieurs éléments consommables (2) pour un système destiné à déterminer une concentration en analytes d'un liquide corporel humain ou animal et est pliée en une pile, les éléments consommables (2) étant disposés en groupes sur la bande porteuse (1), sachant qu'entre des groupes voisins placés dans le sens longitudinal de la bande porteuse (1) il y a un écart plus grand qu'entre les éléments consommables voisins (2) placés à l'intérieur d'un groupe, **caractérisée en ce qu'**une couche de la pile de la bande porteuse (1) contient respectivement au maximum un seul élément consommable (2).

2. Bande porteuse selon la revendication 1, **caractérisée en ce qu'**au moins quelques-uns des éléments consommables (2) sont des éléments de piqûre et/ou des éléments de test ou des éléments de piqûre munis d'éléments de test intégrés.

3. Bande porteuse selon l'une quelconque des revendications précédentes, **caractérisée en ce que** chaque groupe contient au moins trois éléments consommables (2).

4. Bande porteuse selon l'une quelconque des revendications précédentes, **caractérisée en ce que** l'écart entre des éléments consommables voisins (2) est respectivement plus grand que la longueur de la pile, mesurée dans le sens longitudinal de la bande porteuse (1), de préférence au moins deux fois plus grand.

5. Bande porteuse selon l'une quelconque des revendications précédentes, **caractérisée en ce que** l'écart entre des éléments consommables voisins (2) est respectivement plus grand que l'étendue de l'un des éléments consommables (2) dans le sens longitudinal de la bande porteuse (1) plus le double de la longueur de la pile, mesurée dans le sens longitudinal de la bande porteuse (1).

6. Bande porteuse selon l'une quelconque des revendications précédentes, **caractérisée en ce que** l'écart entre des groupes voisins (2) est respectivement plus de deux fois, de préférence plus de trois fois, la longueur de la pile, mesurée dans le sens longitudinal de la bande porteuse (1).

7. Bande porteuse selon l'une quelconque des revendications précédentes, **caractérisée en ce qu'**à l'intérieur des groupes les écarts entre un élément consommable (2) et l'élément consommable identique suivant (2) sont respectivement identiques.

8. Bande porteuse selon l'une quelconque des revendications précédentes, **caractérisée en ce qu'**à l'intérieur des groupes les écarts entre des éléments consommables voisins (2) sont respectivement identiques.

9. Bande porteuse selon l'une quelconque des revendications précédentes, **caractérisée en ce que** les écarts entre des groupes voisins sont respectivement identiques.

10. Bande porteuse selon l'une quelconque des revendications précédentes, **caractérisée en ce que** les éléments consommables (2) dans la pile forment au moins deux piles d'éléments consommables disposées l'une à côté de l'autre.

11. Bande porteuse selon la revendication 10, **caractérisée en ce que** les éléments consommables (2) sont toujours disposés dans différentes piles d'éléments consommables.

12. Bande porteuse selon l'une quelconque des revendications précédentes, **caractérisée en ce que** les couches individuelles de la pile sont de même longueur.

13. Magasin comprenant une chambre dans laquelle est disposée une bande porteuse (1) pliée en une pile, selon l'une quelconque des revendications précédentes.

14. Magasin selon la revendication 13, **caractérisé par** un dispositif d'enroulement (8) destiné à enrouler la bande porteuse (1).
